# EUROPEAN PATENT SPECIFICATION

(11) **EP 1 658 126 B1**
(45) Date of publication and mention of the grant of the patent: **28.04.2010**
(21) Application number: 04743963.3
(22) Date of filing: 15.07.2004
(51) Int. Cl.: B01D 63/02, B01D 69/08, B01D 67/00, A61M 1/16

(54) **SURFACE TREATMENT OF THE MEMBRANE AND ASSOCIATED PRODUCT**
OBERFLÄCHENBEHANDLUNG DER MEMBRAN UND ZUGEHÖRIGES PRODUKT
TRAITEMENT DE SURFACE D'UNE MEMBRANE ET PRODUIT ASSOCIE

(30) Priority: 28.08.2003 FR 0310257
(43) Date of publication of application: 24.05.2006
(73) Proprietor: Gambro Lundia AB, 220 10 Lund (SE)
(72) Inventor: MOACHON, Nicolas, F-38230 Tignieu Jameyzieu (FR); THOMAS, Michel, F-69360 Serezin du Rhône (FR)
(74) Representative: Ponzellini, Gianmarco
(86) International application number: PCT/IB2004/002302
(87) International publication number: WO 2005/021139

(56) References cited:
- EP-A- 0 925 826
- US-A1- 2003 021 826
- US-B1- 6 248 238

## Description

### TECHNICAL FIELD OF THE INVENTION

The purpose of the present Invention is to provide a filtration device, especially useful in the extracorporeal treatment of a fluid such as blood or plasma, a semipermeable membrane mainly consisting of hollow fibers conformed from a unit element consisting of a hollow fiber respectively, said unit element and the manufacturing processes of these objects.

### PRIOR ART

Filtration devices, for the treatment of blood or plasma by extracorporeal circulation, are used in various medical and paramedical applications such as kidney failure treatment by dialysis, ultrafiltration, haemofiltration or haemodiafiltration, therapeutic and non-therapeutic plasmapheresis and apheresis, blood oxygenation, immunoclearance, etc.

### DESCRIPTION OF A FIRST PROBLEM

In patients with renal insufficiency treated for instance by haemodialysis, ultrafiltration, haemofiltration or haemodiafiltratlon by means of a membrane type exchanger, some unwanted reactions called anaphylactoid reactions are observed.

Such reactions may have very severe consequences that can lead to the patients death. Typically, these reactions show up within a few minutes after the beginning of treatment, by various symptoms, such as the sensation of systemic heat, numbness in fingers, lips or tongue, wheezing, nausea, edema of the larynx, etc.

The anaphylactoid reactions were mainly observed during the use of medical devices such as membrane-type exchangers fitted with membranes made of materials with different chemical compositions, whether during single use or multiple uses.

As examples of exchangers the first use of which was accompanied by an unwanted anaphylactoid reaction, there are the dialyzers with polymethylmethacrylate or polyacrylonitrile membrane.

Anaphylactoid reactions associated with the re-use of dialyzers with cellulose acetate and polysulfone membrane were also widely documented (refer to D.A.P. et al., "anaphylactoid reactions associated with the reuse of hollow fibers hemodialyzers and ACE inhibitors" in Kidney International 42, 1232-1237 (1992)).

These anaphylactoid reactions are caused by an excessive concentration, in the blood or the plasma, of a peptidic substance, bradykinin.

An explanation proposed for the generation of bradykinin is summarized below: the blood of patients treated by extracorporeal circulation, that comes into contact with the negatively charged surface of the membranes of the filtration devices, is the medium where a biological phenomenon called "activation of the contact phase" occurs.

This activation, due to the density of negative charges on the surface of said membranes results in the production of active substances such as kallicrein and factor Xlla from inactive substances such as prekallicrein and factor XII, kallicrein having a catalytic effect on the production of factor XIIa and vice versa. But bradykinin results from the transformation by kallicrein - which takes place during the activation of the contact phase - of a plasma protein, the high molecular weight kinogen.

Complications may develop when the activation of the contact phase occurs when, at the same time, some disturbing factors are present in the blood, e.g.
■ drug(s) used in the treatment of hypertension by inhibition of the natural mechanism of vasoconstriction, generically referred to as converting enzyme inhibitors or CEIs; these CEIs are also used for other therapeutic applications, in particular for the treatment of certain forms of cardiac incompetence; but the CEIs are also intended to avoid degradation of bradykinin;
■ diluted blood -this is the dilution of the blood entering a device such as a dialyzer filled with saline solution-and/or a blood pH that is lower than 7.4 -the pH reduction results in the amplification of the reaction of activation of the contact phase.

### STATEMENT OF A SECOND ISSUE

In the case of filtration devices such as dialyzers, a phenomenon called "pressure crossing" is observed. Indeed, in the major part of the filter the pressures are such that the molecules contained in the blood are filtered to the dialysis fluid. However, in a specific area of the filter, the pressures are such that a backfiltration is observed, i.e. certain modules can transit from the dialysis fluid to the blood.

Accordingly, if the dialyzate quality is generally not very critical due to the low porosity of the membrane in filtration devices such as low-flow or medium-flow dialyzers, the same does not apply to high-flow filtration devices, such as streamline high-permeability haemodiafilters where there is a higher risk of backfiltration.

In case of proven backfiltration, - for example in case of poor bacteriological quality of the dialysis fluid -, endotoxins or fragments of endotoxins migrate from the dialysis fluid to the blood. There is a high risk of inflammatory reaction in the treated patient.

### PRIOR SOLUTION KNOWN FOR THE FIRST PROBLEM

Document EP 0.925.826, bearing the Applicant's name, discloses a filtration device for extracorporeal treatment of patient's blood, comprising a negatively charged polyacrylonitrile semipermeable membrane, said membrane being characterized by a surface-limited overall ionic capacity and being treated in the core or in the surface of the membrane with a neutral polymer or a cationic polymer respectively.

Hence, according to this document, such a membrane with such a limited overall ionic capacity may not lead to an activation of the contact phase under normal operating conditions, if the negative charges are neutralized, especially at the membrane surface - since the negative charges might take part in the activation of the contact phase - by combination of said cationic polymer with said membrane.

In the device as disclosed in this document, the treatment only concerns the membrane face that is directly affected by the activation of the contact phase, i.e. the face that will be in contact with the patient's blood.

An inhibition of the contact phase activation is illustrated therein for a hollow fiber dialyzer whose surface area intended to come in contact with the blood is approximately 1.34 m² and is quantified with the flow potential of +2.7 µV/mm Hg by comparison with a untreated dialyzer (for which said flow potential is - 22 µV/mm Hg).

However, recent studies carried out by the Applicant have shown that, in fact, the activation of the contact phase was not fully inhibited.

Indeed, it has been observed that a residual and delayed activation may occur within the first half-hour after the beginning of the treatment. This activation does not occur automatically and it depends on many factors such as, among others, the patient or the filtration device used.

Discomforting clinical symptoms may then occur such as hypertension, diarrhea, etc., even if they are not as severe as those occurring during the immediate contact phase activation.

Therefore, there is a real need for an improved filtration device for extracorporeal treatment of a fluid such as blood or plasma, that is able to overcome the problems encountered in the prior art.

### SOLUTIONS OF THE INVENTION

It has been surprisingly discovered on a filtration device fitted with a negatively charged membrane, that a surface treatment with at least one cationic polymer such as a hydrophilic cationic polymer, preferably water-soluble, or a mixture of polymers of which at least one is a cationic polymer, of such a membrane on the external face of the latter, i.e. the face that will be in direct contact with the filtration or dialysis fluid, enables the retention of endotoxins which otherwise would pass in the blood by backfiltration.

Incidentally, its has also been demonstrated that such a membrane treated on its external face and on its internal face shows a capacity of adsorption of improved heparin, which represents a definite advantage over the non-thrombogenicity of the device fitted with such a membrane.

To the best of the Applicant's knowledge, there is no publication until now that describes an artificial kidney-type filtration device that is fitted with a membrane treated this way on its external face and that unexpectedly results in minimizing the risk of endotoxin backfiltration.

This discovery is the basis of this invention.

Moreover, by implementing this discovery on a membrane of a known filtration device whose external face, intended to be in direct contact with blood, is treated on the surface with at least one cationic polymer such as a hydrophilic cationic polymer, preferably water-soluble, or a mixture of polymers of which at least one polymer is cationic, the Applicant has thus developed new products, such as a membrane and a filtration device, the unit element of which is treated on its two faces, internal and external, and hereafter referred to as "two-face".

Surprisingly these products offer in particular the dual advantage of efficiently avoiding the backfiltration of endotoxins as well the delayed occurrence of the contact phase activation.

In addition to these quite advantageous properties, these products also have a capacity of improved heparin absorption.

### OBJECTS OF THE INVENTION

Therefore, a first object of this invention is a hollow fiber forming a membrane comprising a semipermeable material able to separate in two compartments a filtration device for extracorporeal treatment of a fluid such as blood or plasma, said material being negatively charged, said fiber having a first internal face intended to be in direct contact with blood or plasma and a second external face intended to be in contact with filtrate, characterized in that said second face of said fiber is treated on the surface by at least one cationic polymer or a mixture of polymers of which at least one polymer is cationic. Such an object will be hereafter referred to as "single-face".

Another object of this invention is a hollow fiber as described above the first face of which is also treated on the surface by at least one cationic polymer or a mixture of polymers of which one polymer at least is cationic, hereafter referred to as "two-face",

Still another object of this Invention is a semipermeable membrane comprising an assembly of such hollow fibers.

Still another object of this invention is a filtration device useful for extracorporeal treatment of a fluid such as blood or plasma, comprising two compartments separated by a semipermeable membrane as defined above and mounted in a casing, a first internal compartment being intended for blood or plasma circulation and comprising one or two accesses and a second external compartment being intended for filtrate circulation and comprising one or two accesses, both compartments being also separated by a potting compound, based on an appropriate adhesive compound, intended for making up a sealed partition separating both compartments.

Another object of this Invention is a process for manufacturing a hollow fiber, a membrane and a filtration device such as defined above.

The various objects of this invention and some of their variants will be now presented.

According to one preferred embodiment of the invention, said unit element has the following alternative or complementary features:
■ said membrane material is a homopolymer or an acrylonitrile copolymer;
■ said material of the membrane is a copolymer of acrylonitrile and at least of one non-ionic and non-ionisable monomer, possibly having units from at least another olefinic unsaturated monomer that is capable of being copolymerized with acrylonitrile;
■ said material of the membrane is an acrylonitrile copolymer selected from the group consisting of one acrylonitrile copolymer and at least one anionic or anionisable monomer, possibly enclosing units from at least another olefinic unsaturated monomer that is capable of being copolymerized with acrylonitrile;
■ said at least one anionic or anionisable monomer is an anionic or anionisable olefinic-unsaturated comonomer carrying anionic groups selected from sulfonates, carboxyls, phosphates and sulfates, preferably sulfates;
■ said comonomer is sodium methallysulfonate;
■ said at least one cationic polymer is a hydrophilic polymer selected from the group consisting of a polyamine, a diethylaminoethyldextran (DEAE-Dextran) and a polymer and copolymer containing one or more quaternary ammonium groups;
■ in addition to the already reported preferred features, said material of the membrane has a flow potential greater than zero after sterilization;
■ the hollow fiber can be obtained by a manufacturing process preferably comprising the steps of:
   (i) preparing a polymer solution comprising a material as defined above;
   (ii) extruding the resulting product through a die having two concentric nozzles, a fluid being injected in the internal nozzle to form the aperture of the hollow fiber and then thermomechanically treating (e.g.: stretching) the gel fiber leaving the die; and
   (iii) treating the external face of said hollow fiber by dipping or spraying a solution containing at least one cationic polymer or a mixture of polymers of which at least one polymer is cationic as defined above;
■ said process includes an additional step of treating the Internal face of said hollow fiber with said solution containing at least one cationic polymer or a mixture of polymers of which at least one polymer is cationic.

According to one preferred embodiment, the semipermeable membrane according to this invention has the following feature:
■ It consists of an assembly of unit elements such as hollow fibers to make up a hollow fiber bundle

The negatively charged semipermeable membrane can have an overall ionic capacity - or electrical charge - of less than - 30 µeq/g of membrane.

The device according to the invention has the following alternative or complementary features:
■ said filtration device, useful for extracorporeal treatment of a fluid such as blood or plasma, comprises two compartments separated by a semipermeable membrane mounted in a casing, a first internal compartment being intended for blood or plasma circulation and fitted with two accesses and a second external compartment being intended for filtrate circulation and comprising one or two accesses; both compartments being also separated by a potting compound, based on an appropriate adhesive compound, intended for forming
■ a cylindrical partition separating both compartments of said device containing a semipermeable membrane of the hollow fiber bundle type as defined above.

The process for manufacturing said device of the Invention, useful for extra corporeal treatment of a fluid such as blood or plasma, comprising two compartments separated by a semipermeable membrane mounted in a casing, a first Internal compartment being intended for blood or plasma circulation and fitted with two accesses and a second external compartment being intended for filtrate circulation and fitted with one or two accesses, both compartments being also separated by a potting compound, intended to form a tight separation, is **characterized in that** it comprises a step of circulating, in said external compartment, a polymer solution containing at least one cationic polymer or a mixture of polymers of which at least one polymer is cationic
■ Said process for manufacturing a device according to the invention also comprises a step of circulating, simultaneously or not with the circulation in the external compartment, inside said internal compartment a polymer solution containing at least one cationic polymer or a mixture of polymers of which at least one polymer is cationic.
■ Said process comprises a step of circulating said polymer solution from one end to the other of one of more compartments and then inverting the circulation direction inside the same compartment.
■ Said process also comprises a step of circulating said polymer solution from one end to the other of one of the compartments in one circulation direction.
■ Said process also comprises a step of heparinizing the semipermeable membrane consisting, before use, in pre-heparinizing said membrane, or after mounting, in a heparinizing step during the rinsing of said device.
■ said process comprises a step of gamma-ray sterilization after manufacture of said device.

It is also possible to consider a process for treating a semipermeable membrane not yet inserted in the treatment device as defined above and comprising the same treatment steps as the process for manufacturing the finished device.

### DETAILED DESCRIPTION OF THE PRESENT INVENTION

This invention will now be described in a more detailed manner in its preferred embodiments and using examples of embodiments with reference to the appended drawings in which
Figure 1 illustrates a filtration device according to the "two-face" invention for extracorporeal treatment of blood, of the hemodialyzer/haemofilter type;

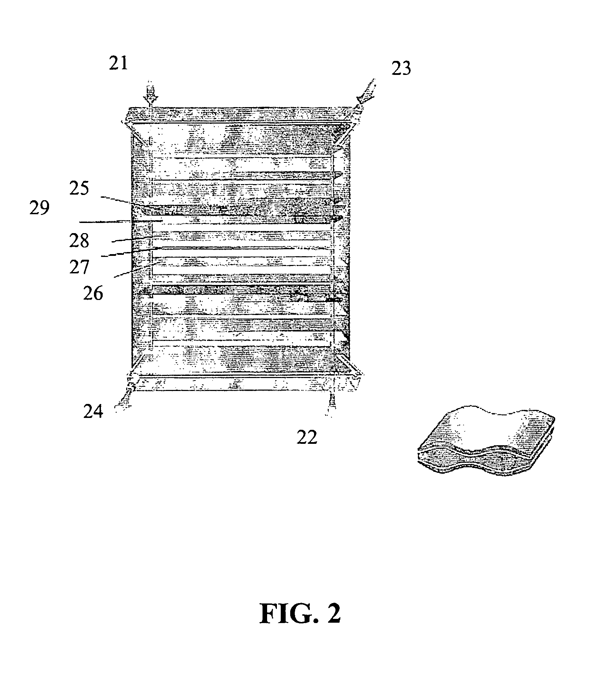

Figure 2 illustrates a semipermeable membrane not according to the invention, of the sheet membrane type made up from unit elements, the sheet films;
Figure 3 illustrates a semipermeable membrane according to the invention, assembled from unit elements, the hollow fibers, to form a hollow fiber bundle;
Figure 4 illustrates the measurement results of the contact phase activation and the finding of a residual and delayed activation of a series of filtration devices whose internal compartment is treated by a process according to the state of the art;
Figure 5 illustrates the measurement results of the contact phase activation and the finding of such an inhibited residual and delayed activation of a series of "two-face" filtration devices according to this invention whose internal and external compartments are treated according to the invention by comparison with a process according to the state of the art; and
Figure 6 illustrates heparin adsorption capacity of a series of filtration devices according to the invention with respect to devices treated according to the state of the art.

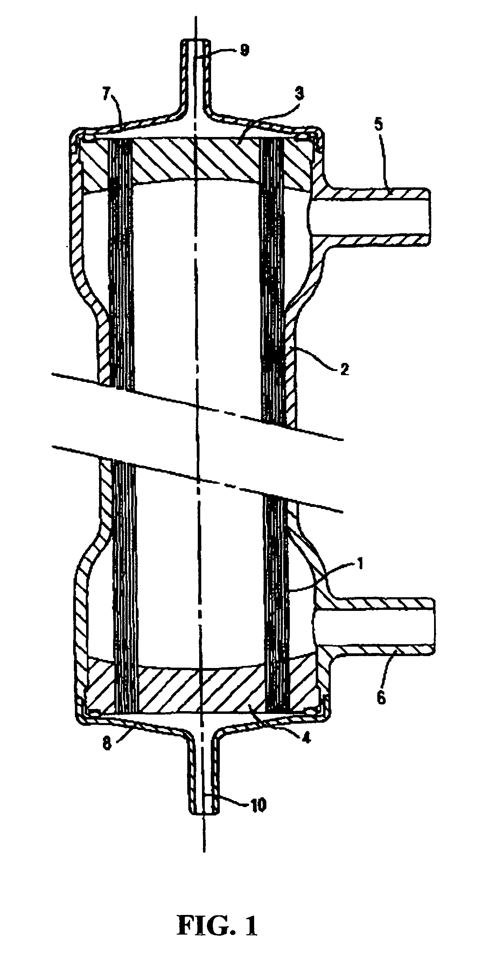

Figure 1 Illustrates a device according to the invention that is an apparatus for extracorporeal treatment of blood useful to compensate for renal insufficiency.

Such an apparatus conventionally comprises two compartments separated by a semipermeable membrane. A first compartment is intended to be connected through a removal line and a return line to the blood-vessel system of a patient respectively referred to as arterial line and venous line, while the second compartment has an inlet possibly connected to a source of fresh dialysis fluid and an outlet connected to a waste fluid drain line.

The membrane is selected to provide for diffusive and/or convective transfers of metabolic end products, from blood compartment to filtrate compartment.

The membrane is manufactured from a unit element such as a hollow fiber.

As can be seen in Figure 1, a filtration device according to this invention comprises a semipermeable membrane such as a hollow fiber bundle (1), which is placed in a tubular enclosure (2) where it is secured at both ends by a disc-shaped partition (3,4). The disc-shaped partitions are not only intended to bind the fibres together, they are also intended to delimit in the tubular enclosure (2) a sealed compartment to which two nozzles (5,6) perpendicular to the centerline of the enclosure (2) give access. To each end of the enclosure (2) is attached and end-fitting (7,8) comprising an axial access nozzle (9,10). Both nozzles (9,10) are symmetric. The blood compartment of the device according to the invention is the inner space between each adhesive disc (3,4) and the end-fitting (8,9) enclosing the corresponding end of the tubular enclosure (2), and from the inside the hollow fibers. The filtrate compartment is the space to which both nozzles (5,6) perpendicular to the centerline of the enclosure (2) give access and comprises the external side of the hollow fibers.

In the scope of this invention:

As a preferred membrane forming material, there is a copolymer of acrylonitrile and sodium methallylsulfonate.

As a preferred cationic polymer useful in the surface treatment according to this invention, there is a high molecular weight polyethyleneimine (PEI).

As other cationic polymers useful in the scope of this invention, there are hydrophilic cationic polymers, highly water-soluble at ambient temperature, such as DEAE-Dextran, and polymers and copolymers containing one or more quaternary ammonium groups.

The "mixture of polymers" preferably consists in the combination of at least one cationic polymer as described above and at least one neutral polymer, highly water-soluble at ambient temperature, such as polyvinylpyrrolidone (PVP), polyethylenegycol (PEG).

When the chemical compound used in the surface treatment is polyethyleneimine (PEI), preferably with an average molecular weight greater than 25,000 Dalton (u), or more preferably greater than 100,000 Dalton (u), it is preferably prepared under the following conditions:
- PEI concentration, from 0.04 g/L to 20 g/L
- medium: water, glycerinated water, salt buffers, salt solutions
- pH: from 7 to 12
- treatment flow rates (case of treatment by circulation in filtration device according to this invention: from 50 ml/min. to 500 ml/min.)
   in the case of a treatment by spraying, the spraying flow rate will be included between 5 and 100 mg/m² (PEI concentration between 0.1 and 30 g/l).
- time: from 1 to 30 min.
- closed or open circuit
- under these conditions, the surface concentration of PEI ranges from 1 mg/m² to 30 mg/m²

In the scope of this invention, the outstanding properties of the objects disclosed therein have been demonstrated using the following test, measurements and/or principles:

In order to show the efficiency of the filtration device of the invention to inhibit the activation of the residual or delayed contact phase, the following test is carried out:

A body fluid, able to stimulate the production of kallicreins (KK) when coming into contact with the membrane negatively charged on the surface, in the sense of the invention is prepared. As a useful liquid for the test, it is possible to use platelet-poor human plasma, diluted 5%. Two liters of this liquid are circulated, in closed circuit at a flow rate of 100 mL/min. in the internal compartment of the device according to the invention.

Plasma kallicreins are measured in the liquid samples taken over time using a conventional chromogenic assay, from substrate S2302 marketed by BIOGENIC.

Thus the increase in kallicrein level (U.K.K/L) over time (min.) is measured in devices according to the invention by comparison with control devices belonging to the prior art.

### Measurement of flow potentials

The flow potential measurements are useful for comparing membranes with the same mass composition but submitted to different surface treatments.

The electrical potential difference is measured across the terminals of a unit element in the sense of the invention, such as a hollow fiber for instance or across the terminals of a membrane in the sense of the invention, especially, a bundle of parallel hollow fibers, in which circulates an electrolyte, such as a 0.01 M sodium chloride solution.

The flow potential represents the ratio between the measured potential difference and the pressure difference applied to the terminals of the unit element (the fiber) or the fiber bundle (the membrane). It is expressed in µV/mmHg.

Thus the flow potentials in the devices according to the invention are measured by comparison with control devices belonging to the prior art (non treated on the surface or only treated on the face in contact with blood).

### Measurement of heparin adsorption capacity

The heparin adsorption capacity is measured during the rinsing of a "two-face" treated device according to the invention and a "single-face on blood side" treated control device belonging to the prior art, with 1 liter of physiological serum containing 5000 UI of non-fractionated heparin with circulation in the internal compartment, "on blood side", open circuit at a flow rate of 150 mL/min.

The capacity of heparin adsorption by the device according to the invention and the control device is assessed by determining heparin concentration in the liquid that circulated in the device (Anti-Xa assay, Stachrom heparin from Diagnostica Stago).

The properties, features and advantages of the objects of the invention will be emphasized in the following non-exhaustive examples only given for reference.

### EXAMPLES

### Example 1:

Conventional filtration device treated according to the state of the art.

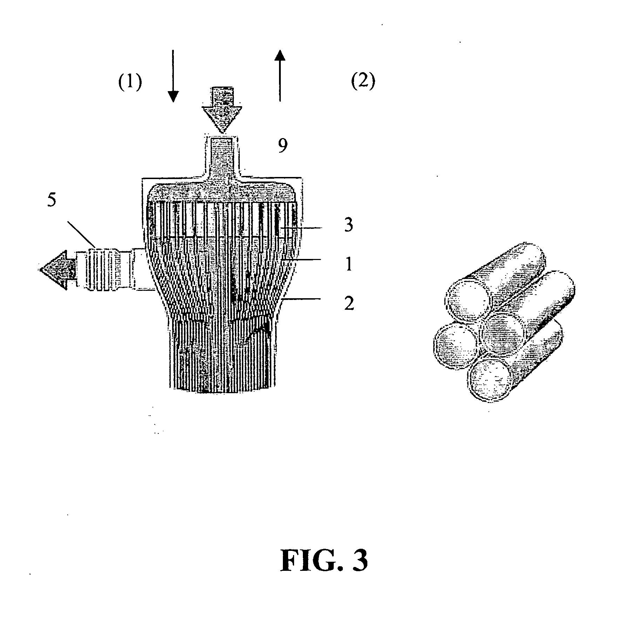

A filtration device such as the hemodialyzer/filter represented diagrammatically in Figure 1 Is manufactured from a membrane of the hollow fiber bundle type as illustrated in Figure 3.

The internal compartment -in contact with blood- is treated with a series of products manufactured from a semipermeable membrane marketed by GAMBRO under "AN69" reference (registered trademark), i.e. made of copolymer of acrylonitrile and sodium methallyl sulfonate. These products are for instance dialyzers called "NEPHRAL" (registered trademark), also marketed by GAMBRO and corresponding to hollow fiber bundles forming AN69 membrane with a useful surface area ranging from about 1 m² to about 2 m². These "NEPHRAL" dialyzers are fitted with hollow fibers with a length between 220 and 280 mm, an inner diameter of about 220 µm, and a thickness of about 42 µm. The treatment process comprises the following steps:
■ water circulation at a flow rate of 150 mL/min for 6 min;
■ identical to the previous example, at a concentration of 0.18 g/L in water and at a pH adjusted to 11 with sodium hydroxide;
■ circulation of the PEI solution in the internal (1) ("blood side") compartment of the device shown in Figure 1 as follows:
   - flow rate of 300 mL/min, for 2.5 min. in direction 1;
   - flow rate of 300 mL/min, for 2.5 min. in direction 2;
■ purging of internal compartment (1) with air: 1 min. at an inlet pressure of 400 mmHg; and
■ sterilization by gamma radiation at about 30 kGy.

### RESULTS

### Measurement of flow potentials

The "blood side" untreated dialyzer referred to as NEPHRAL XT (control) has a flow potential of - 20 ± 2 µV/mmHg, while the dialyzers of the NEPHRAL 300, 400 and 500 series, treated according a process of the state of the art "on blood side" as described in Example 3 have a flow potential of - 5 ± 2 µV/mmHg after sterilization by gamma radiation

### Measurement of the contact phase activation

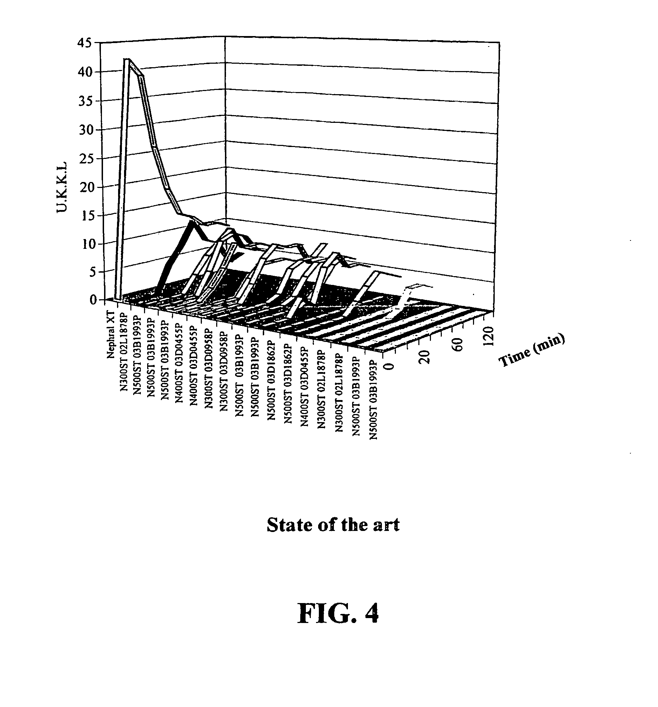

As can be seen in Figure 4, the activation of the contact phase is quantitatively measured with concentration of kallicreins generated as a function of treatment time during the first 2 hours; this applies to several NEPHRAL dialyzers used (N300ST, N400ST and N500ST ranges the series numbers of which are indicated after the range number).

As mentioned above, the activation of the contact phase is delayed because it occurs after the first 20 minutes, but not always since it depends upon several parameters such as the nature of the donor blood for example.

This delayed activation of the contact phase is not quantitatively as severe as the immediate activation in terms of effects on the patient. Indeed, the concentration of the generated kallicreins generated is approximately equal to 10 units.

Thus this example illustrates the activation of the delayed contact phase on the hollow fiber dialyzers according to the state of the art.

### Example 2

### "Two face" filtration device according to the invention

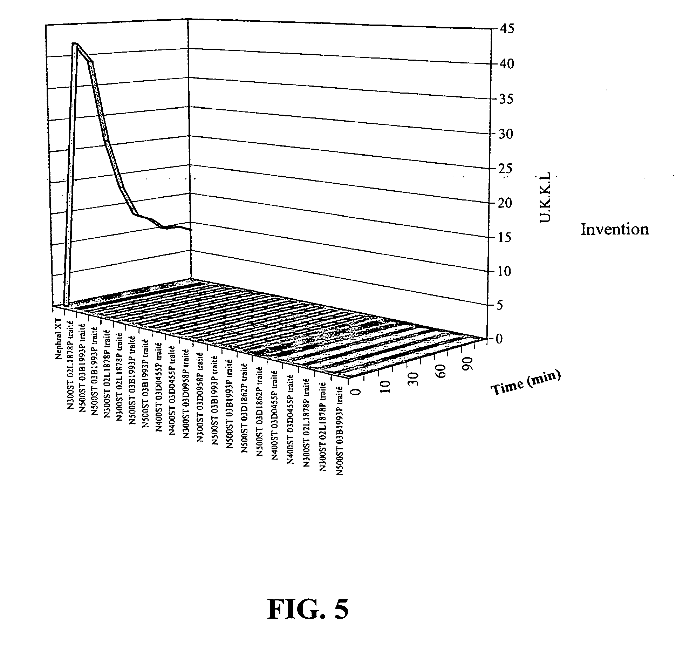

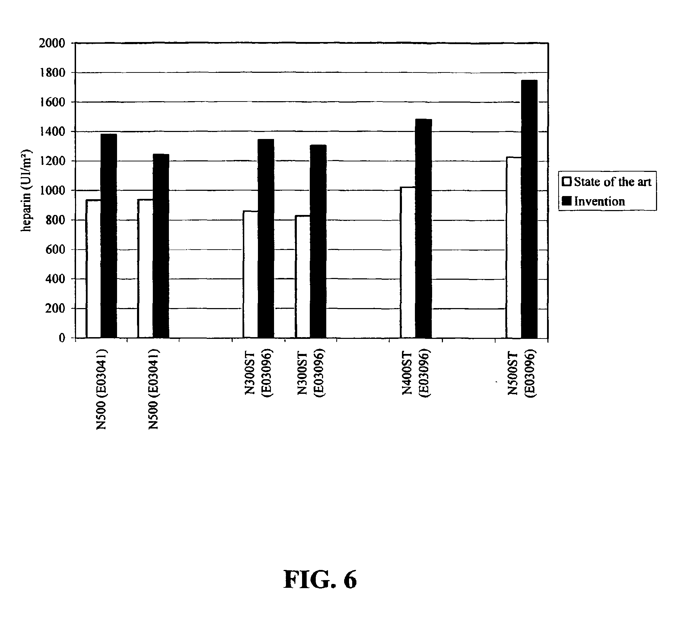

The same products as those used in Example 1 undergo the same treatment, except for the sterilization step, then the treatment continues as follows:
■ treatment with PEI solution as above at a concentration of 0.18 g/L in water and a pH adjusted to 11 with sodium hydroxide In the external compartment ("dialyzate side") of the device as shown in Figure 3;
■ flow rate of 300 mL/min. for 5 min.;
■ purging of the external compartment with air for 1 min. with a pressure of 400 mmHg and
■ sterilization by gamma irradiation at about 30 kGy.
After sterilization, the resulting "two-face" device according to this invention is characterized by the following outstanding properties:
■ A flow potential of + 5 ± 2 µV/mmHg after sterilization by gamma radiation;
■ Full inhibition of the residual and delayed activation of the contact phase (as illustrated in Fig. 5); actually, kallicrein generation is no longer observed, even after two-hour treatment;
■ An improved heparin adsorption capacity (as illustrated in Fig. 6)

## Claims

1. A hollow fiber forming a membrane comprising a semi-permeable material able to separate in two compartments a filtration device for extra corporeal treatment of a fluid such as blood or plasma, said material being negatively charged, the inside of the hollow fiber having a first internal face intended to be in direct contact with blood or plasma and the external side of the hollow fiber having a second external face intended to be in contact with filtrate, **characterized in that** said second face of said hollow fiber is treated on the surface by at least one cationic polymer or a mixture of polymers of which at least one polymer is cationic.

2. Hollow fiber according to claim 1, **characterized in that** said first face is treated on the surface by at least one cationic polymer or a mixture of polymers of which at least one polymer is cationic.

3. Hollow fiber according to claim 1 or 2, **characterized in that** said material of the membrane is a copolymer of acrylonitrile and at least one anionic or ionizable comonomer possibly containing units derived from at least another olefinic unsaturated monomer capable of being copolymerized with acrylonitrile.

4. Hollow fiber according to any one of claims 1 to 3, **characterized in that** said comonomer is an anionic or anionizable comonomer olefinically unsaturated and carrying anionic groups selected from sulfonates carboxyls, phosphates, phosphonates and sulfates.

5. Hollow fiber according to claim 4, **characterized in that** the said material of the membrane is a copolymer of acrylonitrile and sodium methallyl sulfonate.

6. Hollow fiber according to any one of claims 1 to 5, **characterized in that** said at least one cationic polymer is a hydrophilic polymer selected from the group consisting of a polyamine, a diethylaminoethyldextran and a polymer and copolymer containing one or several quaternary ammonium groups.

7. Hollow fiber according to claim 6, **characterized in that** said polyamine is a polyethyleneimine.

8. Process for manufacturing a hollow fiber according to claim 1, comprising the steps of:
(i) preparing a polymer solution containing a material as defined in any one of claims 3 to 5,
(ii) extruding the resulting product through a die having two concentric nozzles, a fluid being injected In the internal nozzle to form the lumen of the hollow fiber and then thermomechanically treating the gel fiber leaving the die and
(iii) treating the external face of said hollow fiber by dipping or spraying a solution containing at least one cationic polymer or a mixture of polymers of which at least one polymer is cationic as defined in claim 6 or 7.

9. Process for manufacturing a hollow fiber according to claim 2, comprising the steps of:
(I) preparing a polymer solution containing a material as defined in any one of claims 3 to 5,
(ii) extruding the resulting product through a die having two concentric nozzles, a fluid being injected in the internal nozzle to form the aperture of the hollow fiber and then thermomechanically treating and stretching the gel fiber leaving the die;
(iii) treating the external face of said resulting hollow fiber by dipping or spraying a solution containing at least one cationic polymer or a mixture of polymers of which at least one polymer is cationic as defined in claim 6 or 7 and
(iv) treating the Internal face of said hollow fiber with said solution containing at least one cationic polymer or a mixture of polymers of which at least one polymer is cationic as defined in claim 6 or 7.

10. Semipermeable membrane consisting of a bundle of hollow fibers according to claim 1 or obtained by assembly of hollow fibers as manufactured by a process according to claim 8 or 9.

11. Filtration device notably useful for extracorporeal treatment of a fluid such as blood or plasma comprising two compartments separated by a semipermeable membrane mounted in a casing (2), a first Internal compartment being intended for blood or plasma circulation and fitted with two accesses (9, 10) and a second external compartment being intended for filtrate circulation and comprising one or two accesses (5,6), both compartments being in addition separated by a potting compound, based on an appropriate adhesive compound, intended for forming
a cylindrical partition for separating the two compartments of said device comprising a semipermeable membrane according to claim 10.

12. A process for manufacturing a filtration device for extracorporeal treatment of a fluid such as blood or plasma comprising two compartments separated by a semipermeable membrane mounted in a casing (2), at first internal compartment for blood or plasma circulation and fitted with two accesses and a second external compartment for filtrate circulation and fitted with one or two accesses, both compartments being in addition separated by the potting compound (3), based on an appropriate adhesive compound, intended to form a tight separation **characterized in that** it comprises a step of circulating, in said external compartment, a polymer solution containing at least one cationic polymer or a mixture of polymers of which at least one polymer is cationic as defined in claim 6 or 7.

13. Process according to the preceding claim **characterized in that** it also comprises a step of circulating in said internal compartment simultaneously or not with the circulation in the external compartment, a polymer solution containing at least one cationic polymer or a mixture of polymers of which at least one polymer is cationic according to claim 6 or 7.

14. Process according to claim 12 or 13 **characterized in that** it comprises a step of circulating said polymer solution from one end to the other of one or two compartments and then inverting the circulation direction inside the same compartment.

15. Process according to claim 12, 13 or 14 **characterized in that** it comprises a step of circulating said polymer solution from one end to the other of one or two compartments in only one circulation direction.

16. Process according to any one of claims 12 to 15 **characterized in that** also comprises a step of heparinizing the semipermeable membrane consisting, before use, in pre-heparinizing said membrane or, after mounting, in heparinizing said membrane during the rinsing of said device.

17. Process according to any one of claims 12 to 16, **characterized in that** it comprises a gamma-ray sterilization step after manufacture of said device.

## Patentansprüche

1. Hohlfaser, die eine Membran bildet mit einem semipermeablen Material, das eine Filtrationsvorrichtung zur extrakorporalen Behandlung eines Mediums wie Blut oder Plasma in zwei Abteile trennen kann, wobei das benannte Material negativ geladen ist, wobei das Innere der Hohlfaser eine erste Innenfläche zur unmittelbaren Berührung mit Blut oder Plasma und das Äußere der Hohlfaser eine zweite Außenfläche zur Berührung mit Filtrat aufweist, **dadurch gekennzeichnet, daß** die benannte zweite Fläche der benannten Hohlfaser durch mindestens ein kationisches Polymer oder ein Polymergemisch, in dem mindestens ein Polymer kationisch ist, oberflächenbehandelt ist.

2. Hohlfaser nach Anspruch 1, **dadurch gekennzeichnet, daß** die benannte erste Fläche durch mindestens ein kationisches Polymer oder ein Polymergemisch, in dem mindestens ein Polymer kationisch ist, oberflächenbehandelt ist.

3. Hohlfaser nach Anspruch 1 oder 2, **dadurch gekennzeichnet, daß** das benannte Material der Membran ein Polymer von Acrylnitril und mindestens einem anionischen oder ionisierbarem Comonomer ist, das gegebenenfalls Einheiten enthält, die von mindestens einem anderen olefinischen ungesättigten Monomer abgeleitet sind, das mit Acrylnitril copolymerisierbar ist.

4. Hohlfaser nach irgendeinem der Ansprüche 1 bis 3, **dadurch gekennzeichnet, daß** das benannte Comonomer ein anionisches oder anionisierbares, olefinisch ungesättigtes Monomer ist, das anionische Gruppen ausgewählt aus Sulfonaten, Carboxylen, Phosphaten, Phosphonaten und Sulfaten trägt.

5. Hohlfaser nach Anspruch 4, **dadurch gekennzeichnet, daß** das benannte Material der Membran ein Copolymer von Acrylnitril und Natriummethallylsulfonat ist.

6. Hohlfaser nach irgendeinem der Ansprüche 1 bis 5, **dadurch gekennzeichnet, daß** das benannte mindestens eine kationische Polymer ein hydrophiles Polymer ist, ausgewählt aus der Gruppe umfassend ein Polyamin, ein Diethylaminoethyldextran und ein Polymer und Copolymer enthaltend eine oder mehrere Quatemärammonium-Gruppen.

7. Hohlfaser nach Anspruch 6, **dadurch gekennzeichnet, daß** das benannte Polyamin ein Polyethylenimin ist.

8. Verfahren zur Herstellung einer Hohlfaser nach Anspruch 1, umfassend die folgenden Schritte:
(I) Herstellung einer Polymerlösung umfassend ein Material wie in irgendeinem der Ansprüche 3 bis 5 definiert,
(II) Extrudieren des erhaltenen Produktes durch eine Matrize mit zwei konzentrischen Düsen, wobei ein Medium in den Innendüse eingespritzt wird, um das Lumen der Hohlfaser zu bilden, und dann thermomechanische Behandlung der die Düse verlassenden Gelfaser, und
(III) Behandlung der Außenfläche der benannten Hohlfaser durch Tauchen oder Spritzen einer Lösung enthaltend mindestens ein kationisches Polymer oder ein Polymergemisch, in dem mindestens ein Polymer kationisch ist, wie in Anspruch 6 oder 7 definiert.

9. Verfahren zur Herstellung einer Hohlfaser nach Anspruch 2, umfassend die folgenden Schritte:
(I) Herstellung einer Polymerlösung umfassend ein Material wie in irgendeinem der Ansprüche 3 bis 5 definiert,
(II) Extrudieren des erhaltenen Produktes durch eine Matrize mit zwei konzentrischen Düsen, wobei ein Medium in den Innendüse eingespritzt wird, um die Öffnung der Hohlfaser zu bilden, und dann thermomechanische Behandlung und Ziehen der die Düse verlassenden Gelfaser;
(III) Behandlung der Außenfläche der benannten Hohlfaser durch Tauchen oder Spritzen einer Lösung enthaltend mindestens ein kationisches Polymer oder ein Polymergemisch, in dem mindestens ein Polymer kationisch ist, wie in Anspruch 6 oder 7 definiert, und
(IV) Behandlung der Innenfläche der benannten Hohlfaser mit der benannten Lösung enthaltend mindestens ein kationisches Polymer oder ein Polymergemisch, in dem mindestens ein Polymer kationisch ist, wie in Anspruch 6 oder 7 definiert.

10. Semipermeable Membran bestehend aus einem Bündel von Hohlfasern nach Anspruch 1 oder erhalten durch Zusammenbau von Hohlfasern hergestellt durch ein Verfahren nach Anspruch 8 oder 9.

11. Filtrationsvorrichtung insbesondere zur extrakorporalen Behandlung eines Mediums wie Blut oder Plasma, umfassend zwei Abteile, die von einer in einem Gehäuse (2) montierten, semipermeablen Membran voneinander getrennt sind, wobei ein erster Innenabteil zur Blut- oder Plasmazirkulation vorgesehen und mit zwei Zugängen (9, 10) ausgestattet ist, und wobei ein zweiter Außenabteil zur Filtratzirkulation vorgesehen ist und ein oder zwei Zugänge (5, 6) umfasst, wobei beide Abteile durch eine Vergussmasse weiter voneinander getrennt sind, die auf Basis von einer geeigneten Klebeverbindung ist, zur Bildung einer zylindrischen Trennwand zur Trennung der beiden Abteile der benannten Vorrichtung umfassend eine semipermeable Membran nach Anspruch 10.

12. Verfahren zur Herstellung einer Filtrationsvorrichtung zur extrakorporalen Behandlung eines Mediums wie Blut oder Plasma, umfassend zwei Abteile, die von einer in einem Gehäuse (2) montierten, semipermeablen Membran voneinander getrennt sind, wobei ein erster Innenabteil zur Blut- oder Plasmazirkulation mit zwei Zugängen ausgestattet ist, und wobei ein zweiter Außenabteil zur Filtratzirkulation mit einem oder zwei Zugängen ausgestattet ist, wobei beide Abteile durch die Vergussmasse (3) weiter voneinander getrennt sind, die auf Basis von einer geeigneten Klebeverbindung ist, zur Bildung einer dichten Trennung, **dadurch gekennzeichnet, daß** es einen Schritt umfasst, worin eine Polymerlösung enthaltend mindestens ein kationisches Polymer oder ein Polymergemisch, in dem mindestens ein Polymer kationisch ist, wie in Anspruch 6 oder 7 definiert, im benannten Außenabteil zirkuliert wird.

13. Verfahren nach dem vorherigen Anspruch, **dadurch gekennzeichnet, daß** es einen Schritt weiter umfasst, worin eine Polymerlösung enthaltend mindestens ein kationisches Polymer oder ein Polymergemisch, in dem mindestens ein Polymer kationisch ist, wie in Anspruch 6 oder 7 definiert, im benannten Innenabteil gleichzeitig oder nicht zur Zirkulation im Außenabteil zirkuliert wird.

14. Verfahren nach Anspruch 12 oder 13, **dadurch gekennzeichnet, daß** es einen Schritt umfasst, worin die benannte Polymerlösung von einem Ende zum anderen Ende eines oder beider Abteile zirkuliert wird und dann die Zirkulationsrichtung in demselben Abteil invertiert wird.

15. Verfahren nach Anspruch 12, 13 oder 14, **dadurch gekennzeichnet, daß** es einen Schritt umfasst, worin die benannte Polymerlösung von einem Ende zum anderen Ende eines oder beider Abteile in einer einzigen Zirkufationsrichtung zirkuliert wird.

16. Verfahren nach irgendeinem der Ansprüche 12 bis 15, **dadurch gekennzeichnet, daß** es einen Schritt weiter umfasst, worin die semipermeable Membran heparinisiert wird, wobei die benannte Membran vor dem Gebrauch vorheparinisiert wird oder nach der Montage während dem Spülen der benannten Vorrichtung heparinisiert wird.

17. Verfahren nach irgendeinem der Ansprüche 12 bis 16, **dadurch gekennzeichnet, daß** es einen Gammastrahlen-Sterilisationsschritt nach der Herstellung der benannten Vorrichtung umfasst.

## Revendications

1. Fibre creuse formant une membrane comprenant un matériau semi-perméable capable de séparer en deux compartiments un dispositif de filtration pour le traitement extracorporel d'un fluide tel que sang ou plasma, ledit matériau étant chargé négativement, l'intérieur de la fibre creuse ayant une première face intérieure apte à être en contact direct avec le sang ou plasma et l'extérieur de la fibre creuse ayant une deuxième face extérieure apte à être en contact avec le filtrat, **caractérisée en ce que** ladite deuxième face de ladite fibre creuse est traitée en surface par au moins un polymère cationique ou un mélange de polymères dont au moins un polymère est cationique.

2. Fibre creuse selon la revendication 1, **caractérisée en ce que** ladite première face est traitée en surface par au moins un polymère cationique ou un mélange de polymères dont au moins un polymère est cationique.

3. Fibre creuse selon la revendication 1 ou 2, **caractérisée en ce que** ledit matériau de la membrane est un copolymère d'acrylonitrile et au moins un comonomère anionique ou ionisable contenant éventuellement des unités dérivées d'au moins un autre monomère oléfinique insaturé pouvant être copolymérisé avec acrylonitrile.

4. Fibre creuse selon une quelconque des revendications 1 à 3, **caractérisée en ce que** ledit comonomère est un comonomère anionique ou anionisable oléfiniquement insaturé et portant des groupements anioniques choisis parmi sulfonates, carboxyles, phosphates, phosphonates et sulfates.

5. Fibre creuse selon la revendication 4, **caractérisée en ce que** ledit matériau de la membrane est un copolymère d'acrylonitrile et méthallyl sulfonate de sodium.

6. Fibre creuse selon une quelconque des revendications 1 à 6, **caractérisée en ce que** ledit au moins un polymère cationique est un polymère hydrophile choisi du groupe comprenant une polyamine, un diéthylaminoéthyldextrane et un polymère et copolymère contenant un ou plusieurs groupements d'ammonium quaternaire.

7. Fibre creuse selon la revendication 6, **caractérisée en ce que** ladite polyamine est une polyéthylèneimine.

8. Procédé pour la production d'une fibre creuse selon la revendication 1, comprenant les étapes suivantes:
(I) préparer une solution de polymères contenant un matériau comme défini dans une quelconque des revendications 3 à 5,
(II) extruder le produit résultant à travers une filière ayant deux buses concentriques, un fluide étant injecté dans la buse intérieure pour former la lumière de la fibre creuse, et ensuite traiter thermomécaniquement la fibre de gel sortant de la filière, et
(III) traiter la face extérieure de ladite fibre creuse par immersion ou pulvérisation d'une solution contenant au moins un polymère cationique ou un mélange de polymères dont au moins un polymère est cationique comme défini dans la revendication 6 ou 7.

9. Procédé pour la production d'une fibre creuse selon la revendication 2, comprenant les étapes suivantes:
(I) préparer une solution de polymères contenant un matériau comme défini dans une quelconque des revendications 3 à 5,
(II) extruder le produit résultant à travers une filière ayant deux buses concentriques, un fluide étant injecté dans la buse intérieure pour former l'ouverture de la fibre creuse, et ensuite traiter thermomécaniquement et étirer la fibre de gel sortant de la filière;
(III) traiter la face extérieure de ladite fibre creuse résultante par immersion ou pulvérisation d'une solution contenant au moins un polymère cationique ou un mélange de polymères dont au moins un polymère est cationique comme défini dans la revendication 6 ou 7, et
(IV) traiter la face intérieure de ladite fibre creuse avec ladite solution contenant au moins un polymère cationique ou un mélange de polymères dont au moins un polymère est cationique comme défini dans la revendication 6 ou 7.

10. Membrane semi-perméable comprenant un faisceau de fibres creuses selon la revendication 1 ou obtenue par assemblage de fibres creuses produites avec un procédé selon la revendication 8 ou 9.

11. Dispositif de filtration utile en particulier pour le traitement extracorporel d'un fluide tel que sang ou plasma, comprenant deux compartiments séparés par une membrane semi-perméable montée dans une carcasse (2), un premier compartiment intérieur étant désigné pour la circulation de sang ou plasma et muni de deux accès (9, 10), et un deuxième compartiment extérieur étant désigné pour la circulation de filtrat et comprenant un ou deux accès (5, 6), les deux compartiments étant en outre séparés par un composé d'empotage à base d'un composé adhésif adapté, apte à former une cloison cylindrique pour séparer les deux compartiments dudit dispositif comprenant une membrane semi-perméable selon la revendication 10.

12. Procédé pour la production d'un dispositif de filtration pour le traitement extracorporel d'un fluide tel que sang ou plasma, comprenant deux compartiments séparés par une membrane semi-perméable montée dans une carcasse (2), un premier compartiment intérieur pour la circulation de sang ou plasma étant muni de deux accès, et un deuxième compartiment extérieur pour la circulation de filtrat étant muni d'un ou deux accès, les deux compartiments étant en outre séparés par le composé d'empotage (3) à base d'un composé adhésif adapté, apte à former une séparation étanche, **caractérisé en ce que** il comprend une étape de faire circuler, dans ledit compartiment extérieur, une solution de polymères contenant au moins un polymère cationique ou un mélange de polymères dont au moins un polymère est cationique comme défini dans la revendication 6 ou 7.

13. Procédé selon la revendication précédente, **caractérisé en ce que** il comprend aussi une étape de faire circuler dans ledit compartiment intérieur, au même temps ou non que la circulation dans le compartiment extérieur, une solution de polymères contenant au moins un polymère cationique ou un mélange de polymères dont au moins un polymère est cationique comme défini dans la revendication 6 ou 7.

14. Procédé selon la revendication 12 ou 13, **caractérisé en ce que** il comprend une étape de faire circuler ladite solution de polymères d'une extrémité à l'autre d'un ou deux compartiments, et ensuite inverser la direction de circulation dans le même compartiment.

15. Procédé selon la revendication 12, 13 ou 14, **caractérisé en ce que** il comprend une étape de faire circuler ladite solution de polymères d'une extrémité à l'autre d'un ou deux compartiments dans une seule direction de circulation.

16. Procédé selon une quelconque des revendications 12 à 15, **caractérisé en ce que** il comprend aussi une étape d'hépariniser la membrane semi-perméable consistant, avant l'usage, à pré-hépariniser ladite membrane ou, après le montage, à hépariniser ladite membrane pendant le rinçage dudit dispositif.

17. Procédé selon une quelconque des revendications 12 à 16, **caractérisé en ce que** il comprend une étape de stérilisation aux rayons gamma après la production dudit dispositif.
